# EUROPEAN PATENT APPLICATION

(11) **EP 2 526 784 A1**
(43) Date of publication of application: **28.11.2012**
(21) Application number: 11167358.8
(22) Date of filing: 24.05.2011
(51) Int. Cl.: A23L 1/29, A23L 1/308, A61K 31/702

(54) **Milk oligosaccharide-galactooligosaccharide composition for infant formula containing the soluble oligosaccharide fraction present in milk, and having a low level of monosaccharides, and a process to produce the composition**

(71) Applicant: Nestec S.A., 1800 Vevey (CH)
(72) Inventor: Berrocal, Rafael, 1806 Saint Légier (CH); Braun, Marcel, 3510 Konolfingen (CH); Cevallos, Agustin, 3532 Zäziwil (CH); Marie, Vanessa, 52037 Sansepolcro (IT); Ricard, Grégoire, 3604 Thun (CH)
(74) Representative: Corticchiato, Olivier

(57) **Abstract**

The invention discloses an oligosaccharide mixture derived from cow's milk that can be easily spray dried comprising (a) a soluble oligosaccharide population which is the same as that of soluble oligosaccharides found in cow's milk and (b) β-galactooligosaccharides formed by the action of β-galacotsidase on lactose and the milk oligosaccharides. The mixture having a total monosaccharide content of less than 5% w/v and a lactose:oligosaccharide ratio of less than 20. A process for obtaining such a mixture, which includes a nanofiltration step, is disclosed. Nutritional compositions, especially infant formula, comprising said oligosaccharide mixture are also disclosed.

## Description

### Field of the invention

This invention relates to an oligosaccharide mixture derived from cow's milk, as well as food products, especially infant formula, comprising said oligosaccharide (OS) mixture and a process for producing said oligosaccharide mixture.

### Background of the invention

The human colon is colonised by a wide range of bacteria having both positive and negative effects on the gut's physiology, as well as having other systemic influences. The predominant groups of bacteria found in the colon include *Bacteroides* species, in particular *Bifidobacteria, Eubacteria, Clostridia* and *Lactobacilli.* These bacteria have fluctuating activities in response to substrate availability, redox potential, pH, O₂ tension and their distribution in the colon. In general, intestinal bacteria can be divided into species exerting either potentially harmful or beneficial effects on their host. Pathogenic effects (which may be caused by *Clostridia* or *Bacteroides,* for example) include diarrhoea, infections, liver damage, carcinogenesis and intestinal putrefaction. Health-promoting effects may be induced through the inhibition of the growth of harmful bacteria, the stimulation of immune functions, improvements in the digestion and absorption of essential nutrients and the synthesis of vitamins. An increase in the numbers and/or activities of bacterial groups (such as *Bifidobacteria* and *Lactobacilli*) that may have health promoting properties is desirable. These "good bacteria" that have beneficial effects on their host are termed "probiotics". Probiotics include many types of bacteria but generally are selected from four genera of bacteria: *Lactobacilllus acidophillus, Bifidobacteria, Lactococcus,* and *Pediococcus.*

Health benefits associated with probiotic bacteria, such as *Lactobacilli* or *Bifidobacteria* include enhanced systemic cellular immune responses, for example, enhanced antibody production and phagocytic (devouring or killing) activity of white blood cells. Certain probiotic bacterial strains have been associated with boosting the immune system thus preventing, or lessening the extent of infection. Some probiotics are associated with allergy prevention as well as the reduction of allergy severity. Several strains have been reported as effective in improvement of intestinal disorders, especially diarrhoea.

Concerning the specific case of infants, immediately before birth, the gastrointestinal tract of an infant is thought to be sterile. During the process of birth, it encounters bacteria from the digestive tract and skin of the mother and starts to become colonised. Large differences exist with respect to the composition of the gut microbiota in response to the infant's feeding. The faecal flora of breast-fed infants includes appreciable populations of bifidobacteria with some *Lactobacillus* species, whereas formula-fed infants have more complex microbiota, with *Bifidobactera species and Bacteroides species, Clostridia* and *Streptococci* being usually present. After weaning, a pattern of gut microbiota resembling that of an adult pattern becomes established.

Mother's milk is recommended for all infants. However, in some cases breastfeeding is inadequate or unsuccessful for medical reasons, or the mother chooses not to breast-feed. Infant formulas have been developed for these situations.

To establish a healthy intestinal bacterial flora, similar to that of breastfed babies, it may be desirable to supplement the infant formula with probiotics. Examples of probiotics currently used in infant formula include *Lactobacillus rhamnosus* ATCC 53103 available from Valio Oy of Finland under the trademark LGG, *Lactobacillus rhamnosus* CGMCC 1.3724, *Lactobacillus paracasei* CNCM I-2116, *Lactobacillus reuteri* sold by BioGaia A.B under the trademark Reuteri, *Lactobacillus johnsonii* CNCM I-1225, *Streptococcus salivarius* DSM 13084 sold by BLIS Technologies Limited of New Zealand under the designation KI2, *Bifidobacterium lactis* CNCM 1-3446 sold *inter alia* by the Christian Hansen company of Denmark under the trademark Bb 12, *Bifidobacterium longum* ATCC BAA-999 sold by Morinaga Milk Industry Co. Ltd. of Japan under the trademark BB536, *Bifidobacterium breve* sold by Danisco under the trademark Bb-03, *Bifidobacterium breve* sold by Morinaga under the trade mark M-16V, *Bifidobacterium infantis* sold by Procter & Gamble Co. under the trademark Bifantis and *Bifidobacterium breve* sold by Institut Rosell (Lallemand) under the trademark R0070.

These probiotics may be administered in amounts of from about one to about twenty billion colony forming units (CFUs) per day for the healthy maintenance of intestinal microflora, preferably from about 5 billion to about 10 billion live bacteria per day. Another factor influencing a healthy intestinal bacterial flora is the presence of prebiotics in the intestine. A prebiotic is an indigestible food ingredient that selectively stimulates the growth and/or activity of the probiotics in the colon, thereby improving the host's health. Prebiotics are indigestible in the sense that they are not broken down and absorbed in the stomach or small intestine, and thus pass intact to the colon where they are selectively fermented by the beneficial bacteria. Examples of prebiotics include certain oligosaccharides, such as fructooligosaccharides (FOS) and galactooligosaccharides (GOS).

Human milk is known to contain a larger amount of indigestible oligosaccharides than most other animal milks. In fact, indigestible oligosaccharides represent the third largest solid component (after lactose and lipids) in breast milk, occurring at a concentration of 12-15 g/l in colostrum and 5-8 g/l in mature milk. Human milk oligosaccharides (HMOs) are highly resistant to enzymatic hydrolysis, indicating that these oligosaccharides may display essential functions not directly related to their caloric value.

Thus, prebiotics act synergistically with probiotics to provide a significant health benefit to the infant. Prebiotics, not only selectively promote the growth of the probiotics that are added to the infant formula, but can also promote the growth of endogenous probiotics capable of acting synergistically with the added probiotics.

Thus, the health benefits provided by probiotics described above are enhanced by the presence of prebiotics.

Thus, as the understanding of the composition of human milk improves, it has also been proposed to add prebiotics to infant formula. These prebiotics are generally administered in amounts sufficient to positively stimulate the healthy microflora in the gut and cause these "good" bacteria to reproduce. Typical amounts are from about one to about 10 grams per serving or from about 5% to about 40% of the recommended daily dietary fiber for the infant.

Thus, various infant formulas supplemented with prebiotics such as mixtures of fructooligosacccharides and galactooligosaccharides, for example, are commercially available.

However, such mixtures provide only an approximation of the mixture of oligosaccharides present in human milk. Over 100 different oligosaccharide components have been detected in human milk, some of which have not yet been detected, or have been detected only in small quantities, in animal milk such as bovine milk. Some sialylated oligosaccharides and fucosylated oligosaccharides are present both in bovine milk and in colostrum, but only in very small quantities.

EP 0 975 235 B1 describes a synthetic nutritional composition comprising one or more human milk oligosaccharides, wherein the HMOs in the composition are chosen among a group of eight HMOs (3-fucosyllactose, lacto-N-fucopentaose III, lacto-N-fucopentaose II, difucosyllactose, 2'-fucosyllactose, lacto-N-fucopentaose I, lacto-N-neotetraose and lacto-N-fucopentaose V), this European patent indicates that, generally speaking, oligosaccharides protect infants from viral and bacterial infections of the respiratory, gastrointestinal and uro-genital tracts.

US Patent Application No. 2003/0129278 describes an oligosaccharide mixture based on oligosaccharides produced from one or several animal milks, characterized in that it comprises at least two oligosaccharide fractions which are each composed of at least two different oligosaccharides. The oligosaccharide population in the oligosaccharide mixture differs from that in the animal milk or animal milks from which the oligosaccharide fractions were extracted.

EP 0 458 358 relates to a process for producing skim milk powder containing 10-15% by weight of galactooligosaccharide, which comprises:
(i) concentrating skim milk to obtain concentrated milk with a solid content of 20- 50% by weight,
(ii) adding ß-galactosidase to the concentrated milk to give rise to an enzymatic reaction,
(iii) heating the resulting reaction mixture for 30 seconds to 15 minutes to a temperature of 70-85°C in order to terminate the enzymatic reaction, and
(iv) spray drying the reaction-terminated mixture.

WO2006/087391 from the present inventors discloses an oligosaccharide mixture derived from animal milk and a process for producing said oligosaccharide mixture. The oligosaccharide mixture is effective as a prebiotic, particularly in the human gut and has an oligosaccharide profile closer to that of human milk than that provided by mixtures of fructo- and galactooligosaccharides.

For the development of prebiotic infant formulas, it is desirable to provide an oligosaccharide mixture that has an oligosaccharide profile as close as possible to that of the source milk, at least in qualitative terms. The relative amounts of oligosaccharides may vary. This means that the oligosaccharide mixture should contain the total oligosaccharide soluble fraction present in milk. By "soluble fraction", it is meant all of the different oligosaccharides that are soluble, generally in milk. The relative amounts of the different soluble oligosaccharides in the source milk need not necessarily be conserved in the oligosaccharide mixture.

Furthermore, it is desirable to maintain a low lactose/oligosaccharide ratio, to avoid introducing unnecessary, high amounts of lactose, and to reduce the amount of additional OS required to attain the desired oligosaccharide level in the infant formula. Also, the oligosaccharide mixture should have a very low protein content so that the amino acid profile of the infant formula is not strongly affected.

An object of the invention is to provide an oligosaccharide mixture which is effective as a prebiotic, particularly in the human gut, and which has an oligosaccharide profile, closer to that of human milk than that provided by mixtures of fructo- and galactooligosaccharides, and having a very low monosaccharide concentration. Thus, the object of the invention is to provide an oligosaccharide mixture containing the oligosaccharide soluble fraction present in milk from which the oligosaccharide mixture is derived. In the context of the current invention, "oligosaccharide mixture derived from cow's milk" means that the oligosaccharides are obtained from cow's milk. Thus, the different soluble oligosaccharides present in the source milk are also present in the final oligosaccharide mixture of the invention, although, not necessarily in the same proportions.

A further object of the invention is to provide an oligosaccharide mixture which has a relatively high oligosaccharide concentration, typically 20-50% w/w.

There is a need for a food product, especially targeted at babies, infants and/or new born infants that helps secure a normal immune or inflammation status, or mitigates or reduces the effect of food allergies.

There is a need to provide a food product which is effective as a prebiotic, particularly in the human gut.

There is a need for a food product that provides the above benefits while preserving a balanced normal metabolism in the individual.

There is a need for an improvement of human gut conditions, by a non-drug-based intervention that is compatible with fragile individuals, like infants or babies.

There is a need for a food product that provides an oral tolerance to allergens.

There is a need for a very low protein milk oligosaccharide ingredient that can be added to infant formula as a syrup or a powder without any carrier.

### Summary of the invention

The current invention relates to an oligosaccharide mixture derived from cow's milk comprising
a. A soluble oligosaccharide population comprising the soluble oligosaccharide fraction found in cow's milk (cow's milk oligosaccharides or CMOS);
b. β-galactooligosaccharides formed by the action of β-galactosidase on lactose and on cow's milk oligosaccharides,
the mixture having a total monosaccharide content of less than 5% w/v, preferably less than 3%, and a lactose:total oligosaccharide ratio of less than 10, preferably less than 3.

The mixture has the following oligosaccharide composition expressed as dry matter percentage
c. Lactose 30-60%
d. Glucose 0.5-2.5%
e. Galactose 0.5-2.5%
f. Oligosaccharide and β-galactooligosaccharides 20-50%
g. Sialyllactose 0.2-2%

Another aspect of the invention is a process for the production of an OS mixture. The steps include
a) concentrating a deproteinised cow's milk material to 50-75% total solids (TS);
b) subjecting the concentrated milk material to a lactose removal step to produce a liquor having a lactose:oligosaccharide ratio of less than 100;
c) optionally clarifying said mother liquor;
d) treating the optionally clarified mother liquor with β-galactosidase to produce a whey material comprising β-galactooligosaccharides;
e) optionally demineralising by, for example, passing the material though a weak cation column and, optionally, a mixed bed column and/or an anion exchange column;
f) carrying out a nanofiltration step, which may be carried out before or after the demineralisation step, and must be carried out after or at the same time as the treatment with β- galactosidase.

The OS mixture of the invention has advantageous properties. It has a glass transition temperature (Tg) in the range of 70-85°C when said mixture has a moisture content of 2.5%. This physical property allows the mixture to be easily spray dried, without caking or sticking , in the absence of carrier. Furthermore, the OS mixture of the invention is less prone to Maillard reactions, compared to non-nanofiltered GOS-containing OS mixtures.

The OS mixture of the invention may be incorporated into a nutritional composition, for example a starter infant formula, an infant formula, a baby food, an infant cereal composition, a follow-on formula or a growing-up milk, preferably a starter infant formula.

Another aspect of the invention is the use of nutritional compositions comprising the OS mixture of the invention for enhancing immune protection and/or reducing the risk of infections and/or reducing the occurrence of food allergies and related food allergy effects on health.

### Brief Description of the Drawings

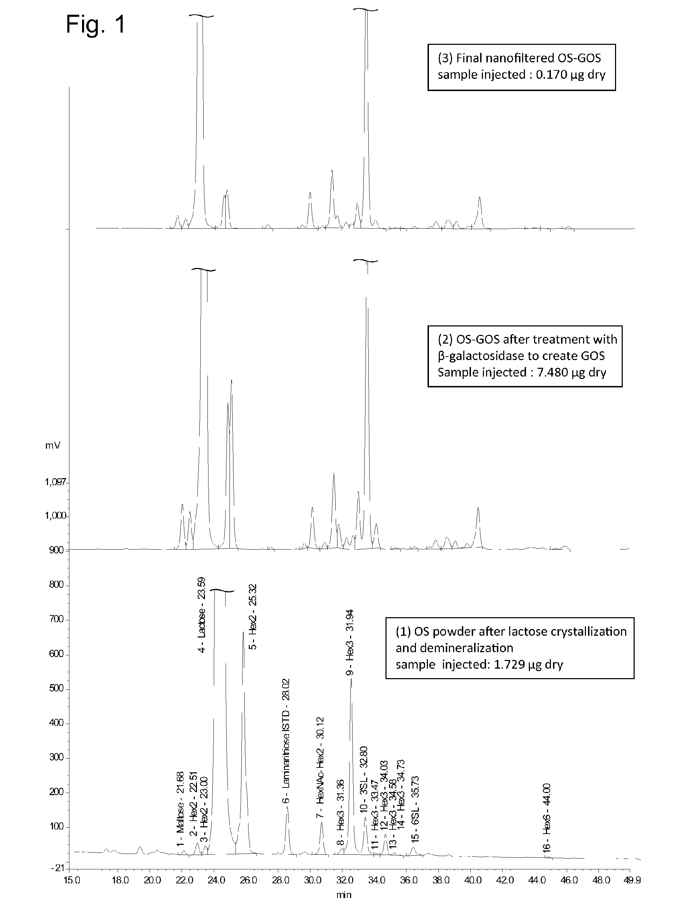

**Figure 1****:** The total soluble oligosaccharide fraction of the cow's milk oligosaccharides (CMOS) and cow's milk oligosaccharides/ß-galactooligosaccharides (CMOS-GOS) mixtures at three points during the process of **Example 1** was determined by HPLC. The HPLC chromatograms correspond to the data obtained for samples of (1) OS powder after partial lactose removal by crystallisation, and demineralisation (2) OS-GOS generated by hydrolysis with ß-galactosidase to create GOS and (3) nanofiltered OS-GOS after nanofiltration (corresponding to Nano OS-GOS in **Example 2**). So that the chromatograms could be read on approximately on the same graphical scale, samples (2) OS-GOS and (3) nanofiltered OS-GOS were diluted 20 and 10 times respectively.
**Figure 2****:**
   The glass transition temperature (Tg) was measured for powders of CMOS-GOS with 30% maltodextrin carrier (in black), CMOS-GOS with 30% lactose (dashed) and the nanofiltered CMOS-GOS mixture according to the invention (in grey). The moisture content of the samples was determined using the Karl Fischer method. It was not possible to obtain values for the glass transition temperatures of CMOS-GOS without carrier because the samples could not be dried to the required humidity level without adding a carrier. However, the data seem to indicate that, without carrier, the Tg value for a non nanofiltered CMOS-GOS would be approximately 35-40°C.
**Figure 3****:**
   The percentages of blocked lysine (due to Maillard reaction) were determined in three infant formulas detailed in **Table 2.** Measurements were made before (grey) and after (black) spray drying. Spray drying was carried out at 92°C. In the graph, the columns "CMOS Vivinal" represent the data for liquid concentrate (grey) and powder (black) infant formulas, to which a non-nanofiltered GOS fraction (Vivinal® GOS from Friesland Campina) and a non-nanofiltered CMOS fraction were added (see **Table 2**). The columns "Nano-CMOS-GOS wet addition" represent the data for infant formulas (liquid concentrate and powder), to which the nanofiltered CMOS-GOS mixture according to the invention is added as a wet mixture. The columns "Nano-CMOS-GOS dry addition" represent the data for infant formula to which the nanofiltered CMOS-GOS mixture according to the invention is added as a dry powder.

### Detailed description of the invention

### Definitions:

As used herein, the following terms have the following meanings.

The term "infant" means a child under the age of 12 months.

The term "young child" means a child aged between one and three years.

The term "infant formula" means a foodstuff intended for particular nutritional use by infants during the first four to six months of life and satisfying by itself the nutritional requirements of this category of person (Article 2 of the European Commission Directive 2006/141/EC of 22 December, 2006 on infant formulas and follow-on formulas).

The term "follow-on formula" means a foodstuff intended for particular nutritional use by infants aged over four months and constituting the principal liquid element in the progressively diversified diet of this category of person.

The term "starter infant formula" means a foodstuff intended for particular nutritional use by infants during the first four months of life.

The term "baby food" means a foodstuff intended for particular nutritional use by infants during the first years of life.

The term "infant cereal composition" means a foodstuff intended for particular nutritional use by infants during the first years of life.

The term "growing-up milk" means a milk-based beverage adapted for the specific nutritional needs of young children.

The term "enhancement of the oral tolerance to allergens" means the reduction of the sensibility to allergens when taken orally.

The term "nutritional composition" means a composition which nourishes a subject. This nutritional composition is usually to be taken orally or intravenously, and it usually includes a lipid or fat source and a protein source.

The term "synthetic composition" means a composition obtained by chemical and/or biological (e.g. enzymes) means, which can be chemically identical to the mixture naturally occurring in mammalian milks.

The term "hypoallergenic composition" means a composition which is unlikely to cause allergic reactions.

The term "oligosaccharide" means a saccharide polymer containing a small number (typically two to ten) of component monosaccharides.

The term "sialylated oligosaccharide" means an oligosaccharide having a sialic acid residue.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J. Nutr. 1995;125:1401-12).

The term "probiotic" means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10).

An "allergy" is an allergy which has been detected by a medical doctor and which can be treated occasionally or in a more durable manner. A "food allergy" is an allergy with respect to a nutritional composition.

The term "oligosaccharide profile" means the identity of a population of oligosaccharides.

All percentages are by weight unless otherwise stated.

### The Oligosaccharides:

Oligosaccharides (OS) are herein defined as those found naturally in animal milks and having a degree of polymerisation (DP) ranging from 3 to 20. These oligosaccharides are soluble in milk. All further references to oligosaccharides in the text refer to soluble (in milk) oligosaccharides unless otherwise stated. The invention provides an oligosaccharide mixture derived from cow's milk wherein the mixture has a lactose (DP = 2):oligosaccharide ratio of less than 20, more preferably less than 10, and preferably between 8 and 1.25. This corresponds to a 4 to 200 times decreased lactose content in the oligosaccharide mixture as compared to the original cow's milk, which is equivalent to a 4 to 200 times increased ratio between oligosaccharides and lactose. Thus, the mixture has the same oligosaccharide profile (i.e. the total soluble oligosaccharide fraction) as that found in the milk from which it was derived (i.e. from which it originated), but with the oligosaccharides at much a more concentrated level.

The oligosaccharide mixture of the invention also contains β-galactooligosaccharides (GOS), resulting from the action of β-galactosidase, mainly on lactose, but also, but to a much lesser extent, on some of the soluble oligosaccharides present in the cow's milk (cow's milk oligosaccharides, or CMOS). The enzyme is added during the production of the oligosaccharide mixture. Exactly which, or to what extent, the CMOS are acted upon by the enzyme has not been determined by the present inventors. Thus, the relative proportions of the different oligosaccharides in the oligosaccharide mixture of the invention may differ from those in the cow's milk from which the mixture is derived. The ß-galactosidase enzyme has a twofold activity: it breaks down lactose into the monosaccharides, galactose and glucose and, secondly, via a transferase activity, it catalyses the subsequent formation of galactooligosaccharides (GOS). These are oligosaccharides are made from glucose and galactose monomers, having a DP of from 3 to 10, and are known to have a prebiotic acitivity.

There are more than twenty different GOS structures present in the oligosaccharide mixtures of the current invention.

The oligosaccharide profile may be characterised by HPLC, Mass Spectrometry and other methods. According to a preferred HPLC method, the oligosaccharides present in the samples are extracted in water at 70°C. The extracted OS are fluorescently labeled by reaction (2h at 65°C) of 2-anthranilic acid amide via formation of a Shiff's base. The double bond is then reduced by reaction with sodium cyanoborohydride to give a stable oligosaccharide aminobenzide (OS-AB) derivative. Labelled extracts are diluted with acetonitrile prior to injection on a HPLC-fluorimeter instrument equipped with a trapping column. Separation is performed on Amide-80 3µm, 4.6x150 mm column, and labeled OS are detected on a fluorimeter at: Ex 330 nm, Em 420 nm. Quantification of the different OS is performed by calibration of the OS-2AB response with maltotriose external standard and using laminaritriose as internal standard.

**Figure 1** shows the total soluble oligosaccharide fraction, as determined by the HPLC method described above, of the mixture at three subsequent steps in the OS mixture production of **Example 1,** (1) CMOS after partial lactose removal by crystallisation, and demineralisation (2) after hydrolysis with ß-galactosidase to create GOS and (3) after nanofiltration (corresponding to Nano CMOS-GOS in **Example 2**). This is the oligosaccharide profile. So that the three chromatograms may be read on approximately the same graphical scale, samples (2) CMOS-GOS and (3) nanofiltered CMOS-GOS were diluted by a factor of 20 and 10, respectively.

The list of oligosaccharides identified from the HPLC data is given in **Table 1**. Not all of the oligosaccharides have been identified, but the profiles in the three chromatograms are very similar. This confirms that the CMOS population of the original cow's milk is, indeed, present in the OS mixture of the invention. The inventors note that the 3'SL peak (corresponding to sialyloligosaccharide 3'sialyllactose) is mainly visible in the chromatogram (1), and not in chromatograms (2) and (3). It is believed that during the ß-galactosidase hydrolysis step of CMOS, one of the GOS created has a retention time very close to that of 3'SL, and this interferes with separation of the 3'SL peak. The inventors have shown that peaks at 32.29 min. (1) and 32.20 min. (2) are not 3'SL, but rather represent a GOS structure (data not shown).

The peak associated with sialyloligosaccharide 6'L (Neu5Ac(α2-6)Gal(β1-4)Glc) in chromatogram (1) is no longer visible in chromatogram (2), due to the dilution factor applied to the CMOS-GOS sample.

**Table 1**

| (1) OS powder from crystallisation and demineralisation | | | |
|---|---|---|---|
| **No.** | **Ret.Time min** | **Peak Name** | **Conc. g/100g** |
| 1 | 21.68 | Maltose | n.a. |
| 2 | 22.51 | Hex2 | 0.0489 |
| 3 | 23.00 | Hex2 | 0.0330 |
| 4 | 23.59 | Lactose | n.a. |
| 5 | 25.32 | Hex2 | 1.0309 |
| 6 | 28.02 | Laminaritriose (int. std) | 0.0000 |
| 7 | 30.12 | HexNAc-Hex2 | 0.1950 |
| 8 | 31.36 | Hex3 | 0.0382 |
| 9 | 31.94 | Hex3 | 1.0213 |
| 10 | 32.80 | 3'SL | 0.2843 |
| 11 | 33.47 | Hex3 | 0.0072 |
| 12 | 34.03 | Hex3 | 0.1100 |
| 13 | 34.58 | Hex3 | 0.0126 |
| 14 | 34.73 | Hex3 | 0.0058 |
| 15 | 35.73 | 6'SL | 0.0781 |
| 16 | 44.00 | Hex6 | 0.0072 |

| (2) OS_GOS after treatment with ß-galactosidase | | | |
|---|---|---|---|
| **No.** | **Ret.Time min** | **Peak Name** | **Conc. g/100g** |
| 1 | 22.04 | Hex2 | 0.2134 |
| 2 | 22.51 | n.a. | n.a. |
| 3 | 23.22 | Lactose | n.a. |
| 4 | 24.88 | Hex2 | 0.6273 |
| 5 | 25.11 | Hex2 | 0.7422 |
| 7 | 29.67 | HexNAc-Hex2 | 0.0318 |
| 8 | 30.15 | Hex3 | 0.2734 |
| 9 | 30.9 | Hex3 | 0.0403 |
| 10 | 31.49 | Hex3 | 0.5278 |
| 11 | 31.81 | Hex3 | 0.1499 |
| 12 | 32.29 | GOS | 0.1065 |
| 13 | 32.69 | Hex3 | 0.0966 |
| 14 | 33.02 | Hex3 | 0.3952 |
| 15 | 33.57 | Hex3 | 2.2039 |
| 16 | 34.15 | Hex3 | 0.1860 |
| 17 | 35.27 | Hex4 | 0.0136 |
| 18 | 36.53 | Hex4 | 0.0206 |
| 19 | 37.85 | Hex4 | 0.1200 |
| 20 | 38.54 | Hex4 | 0.1499 |
| 21 | 39.08 | Hex4 | 0.0667 |
| 22 | 40.5 | Hex4 | 0.4755 |
| 23 | 45.94 | Hex6 | 0.0812 |

| (3) Final nanofiltered OS-GOS | | | |
|---|---|---|---|
| **No.** | **Ret.Time min** | **Peak Name** | **Conc.** g/100g |
| 1 | 21.89 | Maltose | n.a. |
| 2 | 22.38 | Hex2 | 0.4295 |
| 3 | 23.12 | Lactose | n.a. |
| 4 | 24.74 | Hex2 | 1.3178 |
| 5 | 24.92 | Hex2 | 1.5554 |
| 7 | 29.52 | HexNAc-Hex2 | 0.2429 |
| 8 | 30.00 | Hex3 | 2.3388 |
| 9 | 30.74 | Hex3 | 0.1065 |
| 10 | 31.34 | Hex3 | 4.4451 |
| 11 | 32.2 | GOS | 0.4389 |
| 12 | 32.55 | Hex3 | 0.2517 |
| 13 | 32.89 | Hex3 | 1.6449 |
| 14 | 33.44 | Hex3 | 17.2782 |
| 15 | 34.01 | Hex3 | 0.7043 |
| 16 | 35.18 | 6'SL | 0.1053 |
| 17 | 35.67 | Hex4 | 0.0206 |
| 18 | 36.39 | Hex4 | 0.2057 |
| 19 | 37.71 | Hex4 | 0.9629 |
| 20 | 38.42 | Hex4 | 1.2078 |
| 21 | 38.94 | Hex4 | 0.7056 |
| 22 | 39.7 | Hex5 | 0.4053 |
| 23 | 40.37 | Hex5 | 4.5556 |
| 24 | 43.39 | Hex6 | 0.0828 |
| 25 | 43.68 | Hex6 | 0.1968 |
| 26 | 44.41 | Hex6 | 0.1536 |
| 27 | 45.82 | Hex6 | 0.3741 |

| | | | |
|---|---|---|---|
| Hex = Hexose Hex 2 -6 = number of hexoses equivalent to disaccharidses, tri, tetra, penta, hexasaccharides | | | |

The OS mixture of the invention has a total monosaccharide content of less than 5% w/v, preferably less than 3% w/v.

This mixture may be incorporated in infant or adult food products and confers prebiotic, immune modulating and protective effects.

Although the oligosaccharide mixture of the invention is derived from cow's milk, the milk may also be obtained from any kind of animal, in particular from cows, goats, buffalos, horses, elephants, camels or sheep.

### A process for the production of the oligosaccharide mixture:

### Starting Material:

The starting material in the process for producing the oligosaccharide mixture of the invention is a deproteinised milk material, such as milk from which the proteins have been removed, or whey, or any prepared or modified whey material from which the whey proteins have been removed. Such materials include acid whey and sweet whey. Preferred starting materials are milk ultrafiltration permeate and whey ultrafiltration permeate. Alternatively, the starting material may be a reconstituted powder, such as a powdered ultrafiltration permeate.

The starting material must be a deproteinised product because the presence of proteins during concentration can lead to undesirable Maillard reactions and browning. The starting material can be deproteinised by any known means, for example, acid precipitation, heat processes, ion exchange. Preferably, however, removal of protein is carried out by ultrafiltration, which also removes lipids from the starting material.

The pH of the starting material may be between 3 and 7.5, although a pH in the range from 5 to 6 is preferred, to prevent oligosaccharide hydrolysis e.g. desialylation of sialyllactose, and also to help reduce browning reactions.

### a) Concentration of starting material

The deproteinized milk material is concentrated to 50 to 75% total solids (TS), preferably 55 to 60% TS, by any known means, provided that the temperature does not increase to a level which would hydrolyse (e.g. desialylate) the oligosaccharides. Concentration is preferably carried out at temperatures of 50 to 90°C, more preferably 50 to 75°C. Evaporation is one preferred technique, which is carried out at a pressure of from 80 to 200 mbar. In this method, the temperature does not rise above 60°C, which ensures that the oligosaccharides are not adversely affected. Alternatively, if the starting material is a powder, concentration to the desired level may be achieved by appropriate reconstitution of the powder.

### b) Removal of lactose

Preferably, the lactose removal step is carried out by crystallisation and removal of the lactose crystals. Lactose crystallisation may be carried out in the concentrated starting material by cooling the concentrated material with or without addition of a seed crystal, for example. Lactose crystals are then removed by any known method, for example centrifugation, filtration, and/or decantation. An alternative method to separate lactose from the oligosaccharides makes use of differential solubilities. The starting material is spray-dried and then water is added to dissolve the oligosaccharides whilst leaving the lactose in a crystallised form.

The resulting liquor is highly enriched in oligosaccharides, the ratio of oligosaccharides: lactose being 2 to 200 times higher than that found in the milk from which the liquor is derived.

The liquor can be re-concentrated as described above and a further lactose removal step may be carried out. This process may be repeated as often as desired. The final ratio of lactose:oligosaccharides is less than 250, preferably less than 125, and more preferably less than 20.

When the desired amount of lactose has been removed, the liquor may be treated with individual proteases, such as for example trypsin or alcalase, and/or combinations thereof to degrade any remaining milk proteins and peptides into entities with reduced molecular mass. Such a step may be desirable if the mixture is intended for incorporation in a hypoallergenic infant formula.

This step may be carried out according to known methods.

### c) Clarification of mother liquor

This step is optional and may be carried out by any means known to the skilled person, for example, centrifugation.

### d) Treating the deproteinised liquor material with β-galactosidase to produce a liquor material comprising β-galactooligosaccharides (GOS).

Accordingly, the liquor material may be treated with ß-galactosidase before concentration of the milk material (step (a)) and/or after the lactose removal step(s) (step b)). It preferably takes place after completion of the lactose removal step(s). Preferably, the β-galactosidase used is derived from *Aspergillus oryzae.* Such an enzyme is commercially available as Lactase F from Amano, Japan, or Enzeco Fungal Lactase concentrate from Enzyme Development Corporation (EDC), New York, USA. The enzyme activity measured according to the FCCIV method may be between 1,000 and 30,000 U/kg of lactose. The enzymatic treatment may be carried out at a pH in the range from 3 to 7, at a temperature between 4 and 70°C on a starting material with a lactose concentration between 5 and 70 g/100 g total solids (TS) at an enzyme concentration between 0.5-10 g per kg of oligosaccharide mixture.

Preferably, about 1.5 g enzyme is used per kg dry matter of oligosaccharide mixture, and the incubation time is between 1 and 8 hours at 20-70°C. The enzyme may be inactivated after use by application of heat.

After treatment with 0.5 to 6 mg of β-lactosidase per g TS of a liquor having a TS concentration of 25-50% and about 15-40% lactose, the resulting solution may contain about 1 - 4% oligosaccharides, about 9 - 25% GOS, about 15 - 30% lactose, about 5 - 15% galactose and about 2 - 15% glucose. The ratio of oligosaccharides:β galactooligosaccharides (GOS) is preferably in the range from 1:2 to 1:25, more preferably 1:5 to 1:20.

### e) Demineralisation Step:

This step is optional. The liquor may be demineralised by any known means, for example ion exchange, electrodialysis, ultrafiltration or a combination of these processes. The material may be passed through a weak cation column and a mixed bed column and/or an anion column, followed by electrodialysis or nanofiltration, for example. This demineralisation step may be carried out at neutral or acidic pH. It may be carried out before or after the hydrolysis step (d). It also may be carried out in part before hydrolysis and in part after hydrolysis.

### f) Nanofiltration step:

This step is essential to the process of the invention. Nanofiltration of the liquor removes monovalent cations and anions, and monosaccharides. It is desirable to remove monosaccharides from the mixture because (i) they do not have a prebiotic activity and (ii) they induce undesirable reactions with proteins when the prebiotic ingredient is used to produce infant formulas. For example, monosaccharides may lead to Maillard reaction reactivity, wherein lysine residues of proteins are blocked, thus reducing the nutritional quality of the infant formula. Lysine is an essential amino acid that must be provided in the diet, and blocked lysine is not available to the body. Thus, it is desirable to reduce the amount of Maillard reaction occurring during the production of infant formula.

Furthermore, the lower the monosaccharide concentration in the mixture the higher the oligosaccharide concentration can be.

Surprisingly, the inventors have also found that lower monosaccharide levels permit the drying of the oligosaccharide mixture as it is, without addition of carrier. Usually, CMOS-GOS mixtures require the presence of 25 to 35% carrier (such as for example maltodextrin or lactose), to form a powder. The non-requirement of carrier to produce a powder is a considerable advantage in the production and use of the CMOS-GOS mixtures of the invention.

After nanofiltration, the resultant liquor should have a monosaccharide concentration of less than 5% w/v, preferably less than 3% w/v.

Nanofiltration is carried out by passing the liquor through membranes having a pore size small enough to retain oligosaccharides yet large enough to let monosaccharides pass through. For this purpose, commercial membranes with a molecular weight cut-off in the range of 200-1000 Daltons, known in the art, may be used. Among others one may cite, as a non-limitative example of membranes that may used, Nadyr DS NP030 and MMS-LD-3838

The nanofiltration step may be carried out before, or after, the optional demineralisation step. However, the nanofiltration step may also serve to demineralise the liquor. The nanofiltration step must be carried out after or at the same time as the hydrolysis step. If the nanofiltration step is carried out at the same time as the hydrolysis step, the β-galactosidase enzymes may be freely soluble in the retentate tank or immobilised on the nanofiltration membrane.

The nanofiltration step may be combined with a diafiltration step so as to reach the desired monosaccharide content. During the diafiltration step the retentate from nanofiltration is washed several times with an equivalent volume of demineralised water and passed again through the nanofiltration membrane. Typically, the diafiltration step is repeated with 3-5 volumes of water.

After the nanofiltration step, the retentate may contain about 15-30% TS, of which 40-60% is lactose, 0.5-2.5% glucose, 0.5-2.5% galactose, and about 10-50% are oligosaccharides. The ratio of oligosaccharides to β-galactooligosaccharides (GOS) does not change significantly as a result of the nanofiltration step.

The resulting nanofiltered liquor is in a syrup form, and can be used directly as a syrup, or it can be concentrated by evaporation to 74-80% TS, to make it shelf stable, or it can be dried subsequently (e.g. by spray drying) to give a powder. Spray drying methods known in the art may be used.

### Properties of the OS mixture of the invention:

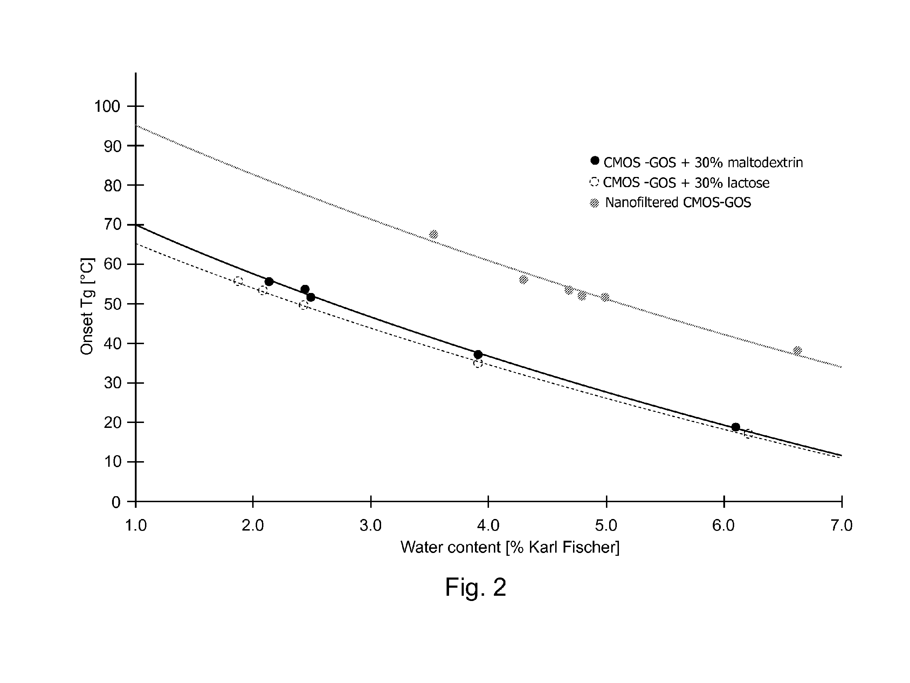

As indicated above, the inventors have found that the process described above produces an oligosaccharide mixture that may be spray dried without carrier. The OS mixtures of the invention have a glass transition temperature in the range of 70-85°C, at a moisture content of 2.5%, as determined by the Karl Fischer method. Oligosaccharide mixtures comprising GOS usually have much lower glass transition temperatures, notably lower than 50°C at this moisture level (see **Figure 2**). Typically, oligosaccharide mixtures having a Tg of around 50°C, or lower, at a moisture content of about 2.5%, require the addition of significant amounts of carrier (typically 15-30%) to allow the mixture to spray dried. Otherwise caking and sticking occur. For example, the powder may stick to the wall of the spray drying tower, thus blocking the dryer. Thus, to form a powder, carrier molecules such as proteins, dextran, maltodextrin, arabic gum, waxy starch and glucose or lactose syrups are generally added.

Thus, the increase in Tg observed for the nanofiltered CMOS-GOS mixtures of the invention allows these mixtures to be successfully spray dried, without caking, in the absence of such carriers.

Thus, the OS mixture of the invention has the key advantage that it may be easily spray dried without the addition of carrier. Thus, one may increase the quantity of oligosaccharide in the final infant formula without introducing undesirable quantities of carrier molecule. This gives the flexibility to deliver the ingredient to factories as liquid or powder, depending on the setup of the factory. The powder production process is simplified.

A similar nanofiltration approach for removing monosaccharides has been tested by the inventors on commercial galactooligosaccharide mixtures. The tests indicate (data not shown) that there is a similar improvement in the drying characteristics of the GOS mixtures. Thus, a commercial GOS syrup that could not be spray dried as is, was successfully spray dried after being subjected to nanofiltration and diafiltration.

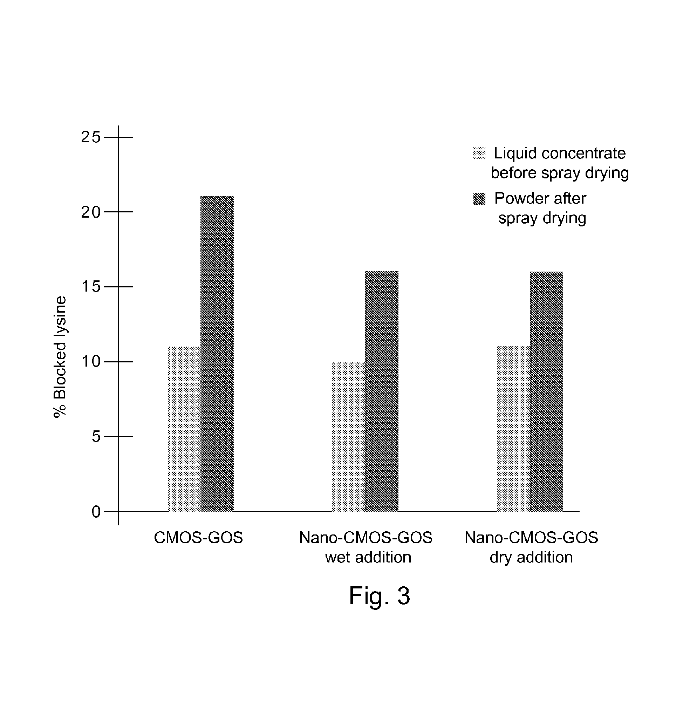

Furthermore, the oligosaccharide of the invention leads to a significant reduction of Maillard reaction reactivity. This is demonstrated in **Figure 3** **(Example 3)** which shows the level of blocked lysine for liquid concentrates and powders of the invention compared to a cow's milk oligosaccharide mixture containing (a) a fraction of non-nanofiltered milk oligosaccharides and (b) a commercial galactooligosaccharide ingredient (Vivinal® GOS, supplied by Friesland Campina). The formulations tested are indicated in **Table 2 (Example 3)**. The non-nanofiltered mixture induces very significant lysine blockage in the final spray dried infant formula. On the contrary, the lysine blockage is much lower if the CMOS GOS fraction has been nanofiltered before the addition to the formula, whatever the form of the addition, in wet or in dry mix. This is due to the removal a substantial proportion of the monosaccharides, which are responsible for the Maillard reaction.

After spray drying, the resulting powder contains approximately 50% lactose and the remainder is a mixture of oligosaccharides (about 20 to 40%, including sialylated oligosaccharides), less than 3% monosaccharides, such as glucose and galactose, about 10% non-protein nitrogen-containing compounds, 3% residual proteins, and some residual salts.

### Uses of the OS mixture of the invention:

In a preferred aspect of the invention, the oligosaccharide mixtures described above are incorporated into a food product. In the context of the present invention, the term "food product" is intended to encompass any consumable matter. Hence, it may be a product intended for consumption by humans, in particular infant formula, dehydrated milk powders including growing-up milks or cereal mixtures.

The infant formula may be prepared in any suitable manner. For example, an infant formula may be prepared by blending together the protein source, any carbohydrates other than lactose and the fat source in appropriate proportions. Emulsifiers may be added if desired. Vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger, e.g. a plate heat exchanger.

The liquid mixture may then be cooled to about 60°C to about 85°C, for example by flash cooling. The liquid mixture may then be homogenised, for example in two stages at about 7 MPa to about 40 MPa in the first stage and about 2 MPa to about 14 MPa in the second stage. The homogenised mixture may then be further cooled to add any heat sensitive components such as vitamins and minerals. The pH and total solids (TS) content of the homogenised mixture is conveniently standardised at this point.

The homogenised mixture is transferred to a suitable drying apparatus, such as a spray drier or freeze drier, and converted to powder. The powder should have a moisture content of less than about 5% by weight.

The oligosaccharide mixture of the invention may be added to the infant formula or other food product by wet mixing at an appropriate stage in the manufacturing process or by dry mixing but is preferably added by wet mixing immediately before the heat treatment and evaporation. However, it will be apparent to the person skilled in the art that the amount of carbohydrate in the infant formula will need to be adjusted to take into account the additional carbohydrate that will be provided by the oligosaccharide mixture. The final concentration of the oligosaccharide mixture in the baby or infant food product or formula is preferably between 2 and 20 g/l, more preferably about 5 g/l of the formula as consumed. However, these amounts should not be considered as limiting and should be adapted to the target population, for example, based on the weight and age or health of the baby or infant. Preferably, the formula containing the oligosaccharide mixture of the invention is fed to the baby at every feed.

Alternatively, the oligosaccharide mixtures may be added to infant or adult food products by dry mixing. The mixture may be added to baby or infant formula at concentrations of from about 1 to 15 grams of oligosaccharides per 100 g of dry formula without bringing unnaturally high amounts of lactose into the formula. However, these amounts should not be considered as limiting and should be adapted to the target population, for example based on the weight and age of the baby or infant, or the health of the specific population.

Although it is preferred to supplement food products specifically targeted towards infant or baby nutrition, it may be beneficial to supplement food products not specifically targeted, or targeted to the adult population. For example, the oligosaccharide mixtures of the invention can be incorporated into healthcare nutrition products and nutritional products for the elderly. Such food products may include milk, yoghurt, curd, cheese, fermented milks, milk-based fermented products, ice-creams, fermented cereal based products, or milk-based products, among others.

The invention will now be further described by reference to the following examples.

### Example 1

Process to prepare the CMOS-GOS mixture of the invention:
207,000 kg of a whey ultrafiltration permeate are pre-concentrated to 29% (w/w) total solids (TS), pasteurised at about 75°C for about 30 seconds and then concentrated by evaporation at 60°C to reach a TS of 58% (w/w). The liquid is distributed in 3 crystallisers and each crystalliser is cooled at a rate of 2°C per hour for a period of 24 hours to crystallise the lactose. Crystallised lactose is washed then removed by a wringer. The remaining liquid is clarified through a decanter.

The 114,000 kg at 23% TS obtained from the clarifier are demineralised by a combination of a weak cation column and a mixed bed column in a manner known per se yielding 109,000 kg of a 90% demineralised liquor at 14.4% TS.
5,400 kg of this demineralised oligosaccharide mixture are concentrated by evaporation to 52% TS. Then it is heated to 60°C in a standard tank at a pH of 5.5 to 6.5. The concentrations of lactose, glucose, galactose, galactooligosaccharides and other oligosaccharides in the mixture are measured. 0.5 g of Enzeco Fungal Lactase concentrate (Enzyme Development Corporation, New York, USA) are added per kg of TS and the mixture is held at 60°C until a glucose concentration of 15% is obtained. Then the temperature is raised to 90°C for 30 seconds by direct steam injection to inactivate the enzyme.
1,500 kg of 52.4% TS hydrolyzed oligosaccharide mixture are diluted with soft water to obtain a 25% TS solution. The solution is standardized in pH 2.5-6.5 and nanofiltered at 10-50°C and 25-30 bars in a 36.25 m² nanofiltration line equipped with MMS-LD-3838 membranes having a nominal cut-off of 300 Daltons. The retentate from nanofiltration is dialfitered with 1 to 5 times its volume with soft water.

The nanofiltered GOS-containing oligosaccharide mix with 22% TS is heat treated at 108°C for 5 s and evaporated to 55% TS. The concentrate is spray dried in an Egron tower using conditions known in the art.

In a second process variant the same nanofiltered GOS containing oligosaccharide mix is heat treated at 108°C for 5 s but evaporated to 74% TS in order to achieve a water activity (a_{w}) lower than 0.86 which renders the concentrated product shelf stable for at least 3 months.

The viscosity of the concentrated product at 74% TS and the ease with which such high concentration level is achieved with the evaporator indicate that a higher concentrations may be reached.

The concentrations of lactose, glucose, galactose, galactooligosaccharides and other oligosaccharides in the mixture are re-measured and the results are shown below in

### Example 2.

### Example 2

This example shows the evolution of the total solids oligosaccharides, monosaccharides, ash, lactic acid and citrate during the process of the invention, in **Example 1**. CMOS corresponds to the mixture before hydrolysis step d), CMOS-GOS corresponds to the mixture after hydrolysis step d), Nano CMOS-GOS corresponds to the mixture after nanofiltration step f). These samples also were used to generate the HPLC chromatograms (1), (2) and (3) in **Figure 1** and data in **Table 1.**

| **w/v %** | **CMOS** | **CMOS-GOS** | **Nano CMOS-GOS** |
|---|---|---|---|
| Total Solids | 98.1 | 52.4 | 97.2 |
| Ash | 1.83 | - | 2.46 |
| Lactic Acid | | - | 0.52 |
| Citrate | 0.34 | - | 0.50 |
| Lactose | 82.7 | 44.05 | 51.14 |
| Glucose | 1.2 | 13.6 | 1.52 |
| Galactose | 4.08 | 6.59 | 1.01 |
| OS +GOS | 1.3 | 17.87 | 36.20 |
| Sialyl-lactose | 0.29 | 0.29 | 0.55 |

### Example 3

Reduction of Maillard reaction reactivity after nanofiltration:
The infant formulas of **Table 2** below were formulated and tested for Maillard reaction reactivity. In the Table, MSW indicates Modified Sweet Whey which is sweet whey from which the caseino-glyco-macropeptide (CGMP) has been removed. Infant formulas comprising the CMOS-GOS mixture of the invention, which was added, either as a liquid concentrate, or as a dry powder, were tested against an infant formula comprising non-nanofiltered CMOS and a commercial GOS (Vivinal® GOS). Standard reaction conditions were used to test for Maillard reaction reactivity. The results are shown in **Figure 3****.** The oligosaccharide mixture of the invention, whether added to the infant formula as a liquid or as a powder, leads to a significant reduction in Maillard reaction reactivity, in the final spray dried product.

**Table 2**

| Nano CMOS-GOS liquid | | Nano CMOS-GOS dry | | CMOS Vivinal® GOS | |
|---|---|---|---|---|---|
| **Wet mix % w/w** | | **Wet mix % w/w** | | **Wet mix % w/w** | |
| Oil mix | 25 | Oil mix | 25 | Oil mix | 25 |
| MSW | 30 | MSW | 45 | MSW | 15 |
| Nano CMOS-GOS | 16 | | | CMOS | 20 |
| | | | | Vivinal® GOS ³ | 7.9 |
| Skimmed milk | | Skimmed milk | 10 | Skimmed milk | 10 |
| LC-PUFAs¹ | | LC-PUFAs | 1.3 | LC-PUFAs | 1.3 |
| Salts | | Salts | | Salts | 1.5 |
| Vitamin premix | | Vitamin premix | | Vitamin premix | 0.3 |
| Lecithin | | Lecithin | | Lecithin | 0.2 |

| **Dry mix** | | **Dry mix** | | **Dry mix** | |
|---|---|---|---|---|---|
| Lactose | 14 | Nano CMOS-GOS ² | 16 | Lactose | 14 |
| Trace element premix | 0.3 | Trace element premix | 0.3 | Trace element premix | 0.3 |
| Probiotics | 0.1 | Probiotics | 0.1 | Probiotics | 0.1 |

| | | | | | |
|---|---|---|---|---|---|
| ¹ Long Chain Polyunsaturated Fatty Acid ² (Example 1 ³ Vivinal® GOS supplied by FrieslandCampina (NL) | | | | | |

### Example 4

An example of an infant formula containing an oligosaccharide mixture according to the present invention is given below. The example is based on a premium whey-predominant Infant formula (from Nestlé, Switzerland) to which the specific oligosaccharides of the invention are added per the amount stated below.

| **Nutrient** | **per 100 kcal** | **per litre** |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE*) | 105 | 700 |
| Vitamin D (µg) | 1.5 | 10 |
| Vitamin E (mg TE**) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| Oligosaccharides CMOS+GOS nano | 2.07 | 13.9 |

| | | |
|---|---|---|
| * RE is Retinol Equivalent ** TE is Tocopherol Equivalent | | |

## Claims

1. Oligosaccharide mixture derived from cow's milk comprising
a. A soluble oligosaccharide population comprising the soluble oligosaccharide fraction found in cow's milk;
b. β-galactooligosaccharides formed by the action of β-galactosidase on lactose and on cow's milk oligosaccharides,
the mixture having a total monosaccharide content of less than 5% w/v and a lactose:total oligosaccharide ratio of less than 10.

2. Oligosaccharide mixture according to claim 1 wherein the mixture has a total monosaccharide content of less than 3%w/v.

3. Oligosaccharide mixture according to claim 1 or 2 wherein the mixture has a lactose:total oligosaccharide ratio of less than 3.

4. Oligosaccharide mixture according to any one of claim 1, 2 or 3 wherein the mixture has the following oligosaccharide composition expressed as dry matter percentage:
a. Lactose 30 -60%
b. Glucose 0.5-2.5%
c. Galactose 0.5-2.5%
d. Oligosaccharide and β-galactoohgosaccharides 20-50%
e. Sialyllactose 0.2-2%.

5. Oligosaccharide mixture according to any one of claims 1-3 wherein the glass transition temperature of the mixture is in the range of 70-85°C when said mixture has a moisture content of 2.5%.

6. Oligosaccharide mixture according to any one of claims 1-3 **characterised in that** it is in the form of a powder and further comprises less than 5% of a carrier molecule.

7. Oligosaccharide mixture according to any one of claims 1-3 **characterised in that** it is in the form of a concentrated syrup with 60-85% total solids (TS), preferably greater than 74% TS.

8. A nutritional composition comprising the oligosaccharide mixture according to any one of the preceding claims.

9. The nutritional composition of claim 9 wherein it further comprises probiotics, preferably wherein the said oligosaccharide mixture promotes the growth or proliferation of said probiotics in the digestive or intestinal tract.

10. A nutritional composition according to claim 8 or 9 that is a starter infant formula, an infant formula, a baby food, an infant cereal composition, a follow-on formula or a growing-up milk, preferably a starter infant formula.

11. Use of the nutritional composition of claims 8, 9 or 10 for enhancing immune protection and/or reducing the risk of infections and/or reducing the occurrence of food allergies and related food allergy effects on health.

12. A process for the production of an oligosaccharide mixture derived from cow's milk comprising the steps
a) concentrating a deproteinised cow's milk material to 50-75% total solids (TS);
b) subjecting the concentrated milk material to a lactose removal step to produce a liquor having a lactose:oligosaccharide ratio of less than 100;
c) optionally clarifying said mother liquor;
d) treating the optionally clarified mother liquor with β-galactosidase to produce a whey material comprising β-galactooligosaccharides;
e) optionally demineralising by, for example, passing the material though a weak cation column and, optionally, a mixed bed column and/or an anion exchange column;
f) carrying out a nanofiltration step, which may be carried out before or after the optional demineralisation step, and must be carried out after or at the same time as the treatment with β- galactosidase.

13. The process of claim 12 wherein step b) comprises a lactose crystallisation step and a concentration step to remove lactose crystals and produce a liquor having a lactose:oligosaccharide ratio of less than 100, these steps being reiterated if necessary.

14. The process of claims 12 or 13 wherein the steps a) to f) are adapted so as to obtain a mixture having a total monosaccharide content of less than 5% w/v, preferably, less than 3% w/v and a lactose:oligosaccharide ratio of less than 20, preferably, less than 10.

15. The process of any of claims 12 to 14, wherein said process is adapted to obtain the mixture of any one of claims 1 to 7.

16. The process of any of claims 12 to 15 wherein the deproteinised cow's milk material is a milk ultrafiltration permeate or a whey ultrafiltration permeate.

17. The process of any one of claims 12-16 further comprising the treatment of the liquor product with individual proteases and/or combinations thereof to degrade any remaining milk proteins and peptides into entities with reduced molecular mass.

18. The process of any one of claims 12 to 17, wherein the β-galactosidase used is derived from *Aspergillus oryzae.*

19. The process of any one of claims 12 to 18, wherein the nanofiltration step is combined with 1-5 diafiltration steps.

20. The process of any one of claims 12 to 19 further comprising the step of spray-drying the liquor product to give a powder.

21. The process of claim 20 wherein less than 5% carrier, preferably 0% carrier, is added to the mixture during the step of spray drying.

22. A product obtainable by the process of any one of claims 12 to 21.
